# EUROPEAN PATENT SPECIFICATION

(11) **EP 4 183 370 B1**
(45) Date of publication and mention of the grant of the patent: **21.10.2026**
(21) Application number: 22208739.7
(22) Date of filing: 22.11.2022
(51) Int. Cl.: A61F 2/24

(54) **DEVICE WITH A TEMPORARY VALVE FOR PERCUTANEOUS DELIVERY IN A NATURAL OR ARTIFICIAL AORTIC VALVE**
VORRICHTUNG MIT EINER PROVISORISCHEN KLAPPE ZUR PERKUTANEN LIEFERUNG IN EINER NATÜRLICHEN ODER KÜNSTLICHEN AORTENKLAPPE
DISPOSITIF AVEC VALVE TEMPORAIRE POUR ACCOMPAGNEMENT PERCUTANÉ DANS UNE VALVE AORTIQUE NATURELLE OU ARTIFICIELLE

(30) Priority: 23.11.2021 IT 202100029579
(43) Date of publication of application: 24.05.2023
(73) Proprietor: Leafmate S.r.l., 25122 Brescia (IT)
(72) Inventor: CURELLO, Salvatore, 25122 Brescia (IT); MAALOUF, Mirna, 25122 Brescia (IT)
(74) Representative: Gualeni, Nadia

(56) References cited:
- EP-A1- 3 495 012
- WO-A1-2018/078563
- WO-A2-2006/031648
- WO-A2-2012/009558

## Description

The present invention generally relates to a device for performing procedures on natural or prosthetic heart valves, particularly during the replacement of these valves.

Heart valves, such as the aortic valve, are sometimes damaged by disease or aging which may make them stop working properly. In severe cases, it becomes necessary to replace the valve to restore heart function. In common practice, the replacement requires open heart surgery.

An alternative methodology is percutaneous valve replacement with a beating heart. Transcatheter aortic valve implantation/replacement (TAVI or TAVR) has become very popular because it avoids the potential complications of opening the chest and placing the patient on cardiopulmonary bypass. This procedure requires the new valve (Transcatheter Aortic Valve - TAV) to be placed via a catheter passed through the blood vessels, or through the apex of the heart into the ventricular cavity. These valves consist of tissue leaflets mounted on a frame that is expanded and anchored in proximity to the existing diseased native valve. It should be noted that even the replacement valve (TAV) does not have an unlimited life and must be replaced again after 5-10 years. In this case, a particular procedure is envisaged, called TAV-in-TAV, which consists in inserting a second TAV inside the first damaged TAV. This procedure is also possible in the case of surgical bioprosthetic valves (Surgical Aortic Valve - SAV). In this case, the procedure is called TAV-in-SAV, which consists in inserting a TAV valve inside a damaged SAV. At the end of the TAV-in-TAV or TAV-in-SAV procedure, the damaged leaflets of the first valve are vertically compressed between the frame of the first and second TAV.

In certain aortic anatomy, it would be necessary to remove the leaflets of the first TAV or SAV, to prevent their presence from impeding the correct perfusion of the right and/or left coronary arteries. There is also the need to remove the native leaflets because they are infected or potentially obstructive on the coronary arteries during the implantation of the first TAV. The challenge of removing the leaflets is related to the need to perform the procedure quickly. At the time the valve is excised, the patient is essentially valveless and therefore without normal cardiac output flow. In fact, the patient no longer has a functioning valve and is subject to severe aortic regurgitation. In other words, blood is free to return to the ventricle during diastole, as it is no longer blocked by the leaflets of the first valve. Therefore, in the absence of the valve, the diastolic pressure inevitably tends to drop below a level that does not guarantee correct coronary perfusion, causing the patient's hemodynamic instability.

To solve this problem, solutions are known which provide for the placement of a temporary valve to increase blood pumping while the damaged valve is in the process of being repaired, removed or replaced with a new valve.

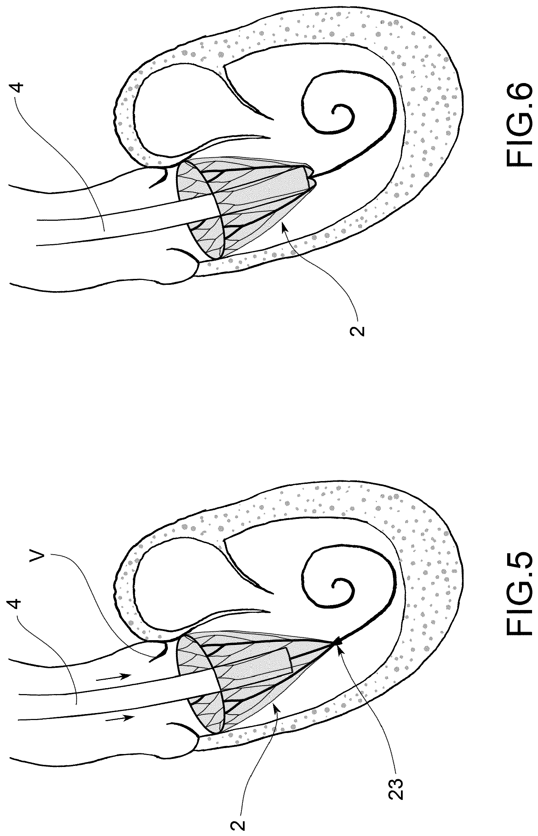

In the solution described in EP 1 472 996 B1, the temporary valve is positioned in the ascending aorta, above the aortic valve. This position does not allow diastole blood flow to perfuse the coronary arteries, as retrograde flow would be inhibited. Furthermore, as may be seen in Fig. 5A, the positioning device of the temporary valve does not allow positioning of the TAV, which in fact requires an additional guide to reach the ventricle. Furthermore, this device provides for the TAV to pass laterally to the temporary valve, thus interfering with the opening and closing thereof. The same applies to the solution described in EP 3 403 615 A1, in which the temporary valve is positioned in the ascending aorta, above the aortic valve. In both of these solutions the valve is not, and may not, due to its construction, be turned inside out.

In the solution described in document WO 2012/021527 A2, the temporary valve is positioned in the ventricle below the aortic valve via a positioning catheter provided with an internal lumen for the passage of further instruments. This catheter is inserted through a puncture into the ventricle, as shown in Fig. 11. This document also mentions the possibility of the delivery catheter being passed through the aortic valve. However, it should be noted that in this second hypothetical solution the delivery catheter would be inside the damaged aortic valve, itself preventing the implantation of the new TAV valve. Furthermore, it is not clear how the new TAV valve may be deployed since there are no outlet ports along the delivery catheter. A similar solution is proposed in the document WO 2015/123607 A2. In both of these solutions the valve is not, and may not, due to its construction, be turned inside out. Other examples of temporary valves are disclosed in documents EP 3 495 012 A1 and WO 2018/078563 A1.

It is an object of the present invention to provide a device with a temporary valve to be positioned just below the damaged heart valve (be it a natural valve, a SAV valve or a TAV valve), i.e. inside the left ventricle, so that it performs the role of the aortic valve in the process of removing or cutting the damaged heart valve leaflets and implanting the replacement TAV.

Furthermore, it is an object of the present invention to provide a device with a temporary valve which serves both as a temporary valve and as a guide for implantation of the replacement TAV.

Furthermore, it is an object of the present invention to provide a device with a temporary valve which also acts as a guide for the use of devices for cutting or removing the leaflets.

Furthermore, it is an object of the present invention to provide a device with a temporary valve which is minimally invasive and easily removable without the risk of damage to the newly positioned replacement TAV.

This object is achieved by means of a device with a temporary valve according to claim 1. The dependent claims describe preferred embodiments of the invention.

The features and advantages of the device with a temporary valve according to this invention will appear more clearly from the following description, made by way of an indicative and non-limiting example with reference to the accompanying figures, wherein:
- Fig. 1 and 2 show the device with a temporary valve being inserted into the left ventricle; in particular, Fig. 1 shows the temporary valve completely contained inside the sheath and Fig. 2 shows the temporary valve completely open below the aortic valve;
- Fig. 3 and 4 show the device with a temporary valve in the ventricle in the operating step; in particular, Fig. 1 shows the temporary valve in the diastole phase and Fig. 2 shows the temporary valve in the systole phase;
- Fig. 5 to 8 show the device with a temporary valve according to the present invention being removed from the ventricle; in particular, Fig. 5 shows the advancement of the sheath, Fig. 6 shows the withdrawal of the temporary valve which, turning inside out, re-enters the inside of the sheath, Fig. 7 shows the further withdrawal of the temporary valve now almost completely turned inside out and reinserted into the sheath, and Fig. 8 shows the final withdrawal with the temporary valve completely turned inside out and reinserted into the sheath;
- Fig. 9 and 10 show the placement of a TAV replacement valve by means of the device with a temporary valve in the ventricle during operation; in particular, Fig. 9 shows the advancement of the TAV delivery catheter on the device according to the present invention, and Fig. 10 shows the delivery of the TAV over the damaged valve while the temporary valve according to the present invention continues its operating step;
- Fig. 11 shows a detail of the device with a temporary valve, and in particular the frame of the temporary valve;
- Fig. 12 shows a detail of the device with a temporary valve, and in particular the fixing of the temporary valve on the guide body;
- Fig. 13 shows a detail of the device with a temporary valve, and in particular the tip of the guide body.

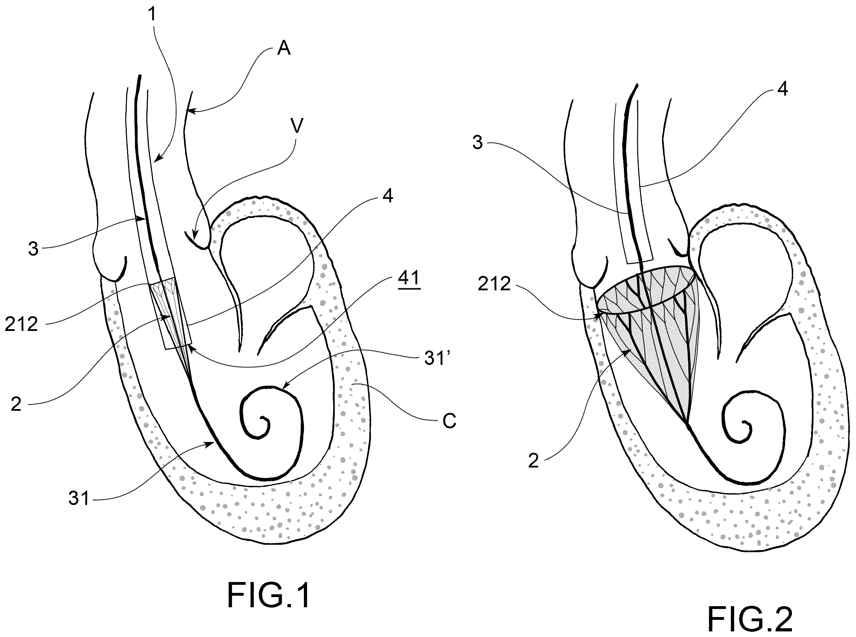

Fig. 1 shows a section of a heart portion C, and in particular the left ventricle, in which there is a defect of the aortic valve V, whether it is a natural valve or a first replacement valve TAV.

The device 1 comprises a temporary valve 2 fastened to a support body 3.

Preferably, the support body 3 is provided with a flexible, preformed tip 31, provided with a final curvature or curl 31'.

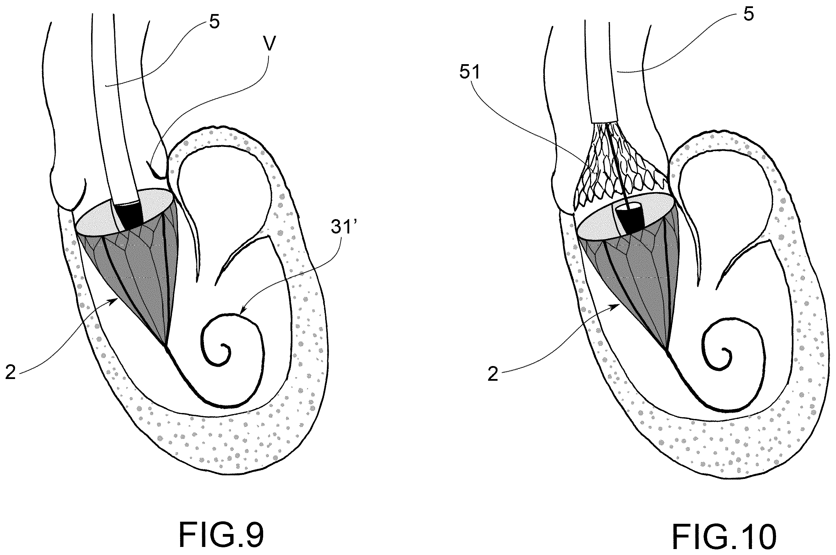

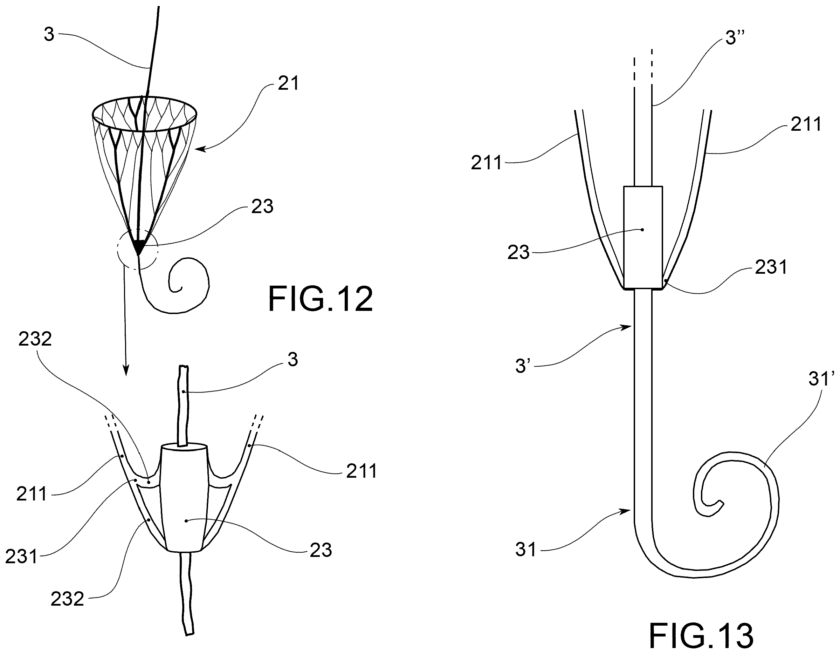

In an embodiment, shown in Fig. 12, the support body 3 is a solid body, preferably a guide wire. The support body 3 therefore has no internal lumens to maintain reduced dimensions and to be able to act as a guide wire for further devices, such as for example for a delivery catheter 5 of a replacement valve 51 as shown in Fig. 9 and 10.

In this example, the support body 3 is a guide wire with a metal core (for example made of stainless steel) and coating made of a hydrophilic material (for example polytetrafluoroethylene - PTFE). Preferably, the support body 3 has a diameter of 0.035" (inches) or 0.889 mm.

Preferably, the support body 3 has a length of more than 230 cm (including the curled tip).

Preferably, the support body 3 is a rigid body with a flexible curled tip 31. The flexibility of the tip 31 is obtained by a gradual thinning of the metal core.

In a further embodiment, shown in Fig. 13, the support body 3 is composed of an external sheath 3" inside which a solid internal body 3' slides coaxially. In this example, the outer sheath 3'' ends distally, and is connected, at the apex 23 of the frame 21 of the temporary valve 2. The solid internal body 3' ends distally, forming the flexible tip 31. By advancing the solid internal body 3' in the external sheath 3'' it is possible to lengthen the flexible tip 31, and in particular the distance between the apex 23 of the frame 21 and the curled part of the flexible tip 31. The curled end of the flexible tip 31 is in fact made to come into contact with the apex of the ventricle, which, depending on the patient's anatomy, may be more or less deep, as seen in Fig. 1. This embodiment allows the operator to push the curled part up to the apex of the ventricle without interfering with the positioning of the temporary valve 2.

In this example, the support body 3 comprises the external sheath 3'' having a diameter of 0.035" (inches) or 0.889 mm and lined with hydrophilic material, inside which there is the solid metal internal body 3' with a smaller diameter, which forms the curled tip in its distal part.

As mentioned above, the support body 3 is provided with a curled flexible tip 31. Preferably, the tip has a length of between 15 and 30 cm and is curled on itself to form a spiral or a curve with a width/diameter of between 2 and 8 cm. The tip is also composed of a metal core and a coating of hydrophilic material. The curled tip is less rigid than the body of the support body 3 and this rigidity is obtained by a gradual thinning of the metal core.

In an embodiment, as in Fig. 12, the flexible tip 31 is the terminal end of the support body 3 and is made integrally therewith.

The device 1 further comprises a sheath 4 slidably mounted along the support body 3 and adapted to keep the temporary valve 2 in the collapsed configuration so that it may be moved through the body lumen to the point of use.

Fig. 1 shows the temporary valve 2 in the operating configuration, and therefore open and placed against the walls of the outflow tract of the left ventricle following the withdrawal of the sheath 4.

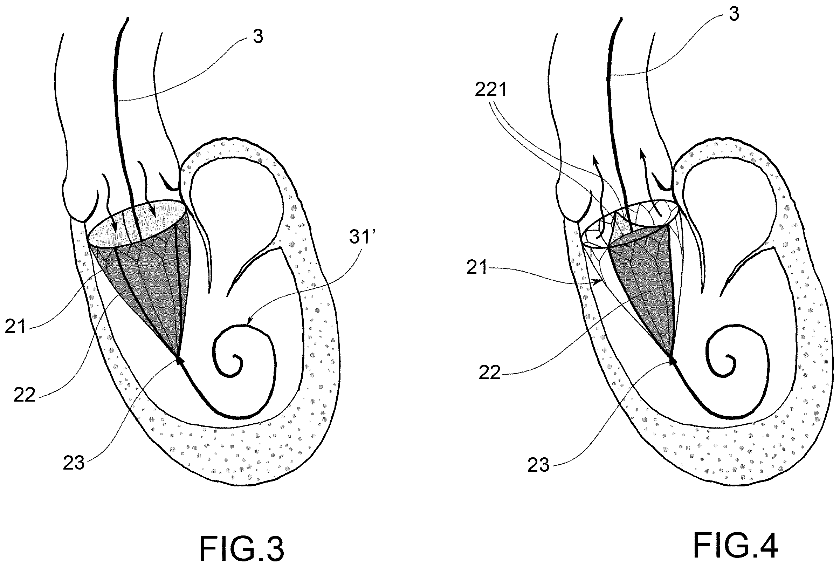

Fig. 3 shows the temporary valve 2 in the closed blood flow state, with the membrane 22 open against the frame 21, in the diastole phase, to prevent the blood flow through the valve. Fig. 4 shows the temporary valve 2 in the open blood flow state, with the membrane 22 closed towards the support body 3, in the systole phase, to allow blood flow through the valve. The temporary valve 2 passively alternates between the open state and the closed state in response to the pressure acting thereon.

The temporary valve 2 comprises a frame 21 and a membrane 22 fastened to the frame 21. The frame 21 allows the temporary valve 2 to pass between the collapsed configuration (Fig. 1, 6, 7 and 8) and the operating configuration (Fig. 2, 3 and 4, 5, 9 and 10). When in the operating configuration, the membrane 22 allows the temporary valve 2 to pass between the closed state (Fig. 3) and the open state (Fig. 4).

As may be seen in the figures, the temporary valve 2 is an umbrella valve provided with an apex 23 oriented in the distal direction. The temporary valve 2 is fastened to the support body 3 at the apex 23 so that the distal end of the temporary valve 2 points towards the heart.

Preferably, the frame 21 is made of shape memory metal, for example nitinol. The frame 21 is self-expandable due to the shape memory material.

Preferably, the frame 21 is provided with at least one radiopaque marker placed for example at the annular edge 212.

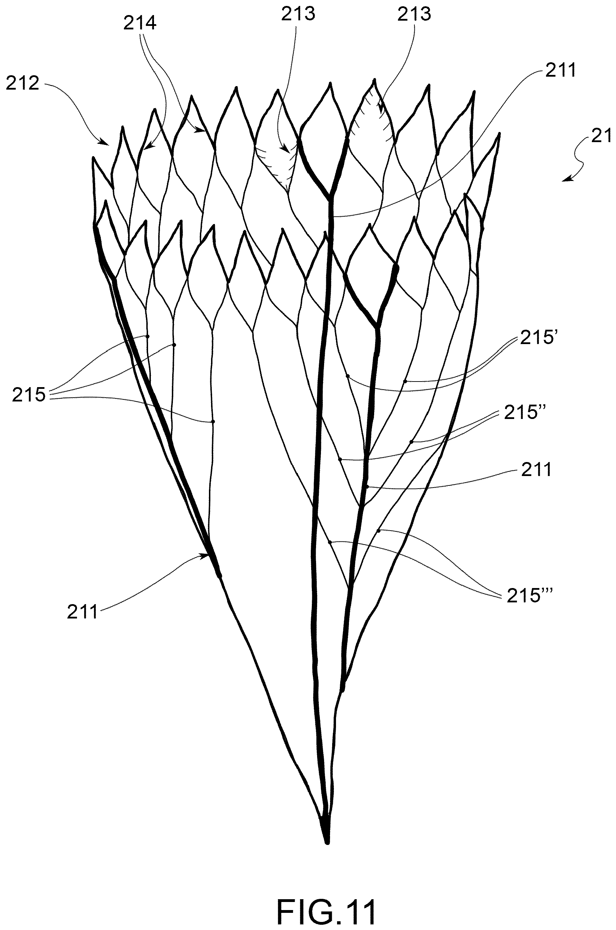

Preferably, as may be seen in Fig. 11, the frame 21 comprises an annular edge 212 from which main uprights 211 start, which uprights are arranged equidistant from each other and connected to each other at the apex 23.

When the temporary valve 2 is in the collapsed configuration, as in Fig. 1, the annular edge 212 is also in the collapsed configuration and, given the accumulation of material, itself performs the function of marker without the need for added radiopaque markers.

When the temporary valve 2 is in the operating configuration, as seen in Fig. 2, the annular edge 212 rests against the wall of the outflow tract of the left ventricle, just below the aortic valve V to be treated. Preferably, the frame 21 is provided with anchoring elements 213, for example teeth or hooks, at the annular edge 212 to improve the maintenance of the temporary valve 2 in position in the operating configuration.

The annular edge 212 is wavy, or jagged, or is a continuous ring.

In a preferred example, the annular edge 212 is formed by a continuous succession of cells 214. In one example, the cells 214 are closed cells, for example diamond-shaped and connected to each other at the smaller diagonal. In another example, the cells 214 are open cells, for example V or U-shaped, with the vertex facing the apex 23 and connected to each other at the upper ends.

Preferably, the frame 21 comprises at least three main uprights 211.

Each main upright 211 has increasing flexibility towards the apex 23, i.e. the upright has progressively greater flexibility in the distal direction. This flexibility is obtained for example through a thinning of the section of the main upright 211, thinning increasingly more towards the apex 23.

Preferably, as may be seen in Fig. 11, the frame 21 also comprises a plurality of secondary uprights 215, each of which branches off from the annular edge 212 and connects to a main upright 211.

The secondary uprights 215 are shorter, thinner and/or more flexible with respect to the main uprights 211.

The secondary uprights 215 are arranged as branches branching off from a main upright 211, or as the veins of a leaf. The secondary uprights 215 differ from each other according to the distance from the main upright 211: the closest secondary upright is shorter than the following secondary upright.

In one example, each secondary upright 215 connects to a different point of the relevant main upright 211 with an offset trend between right and left.

In an example, the secondary uprights 215 are arranged in pairs, with a first secondary upright of the pair arranged on one side of the main upright 211, and a second secondary upright of the pair arranged specularly on the opposite side of the main upright 211. The pair 215' of secondary uprights forms a V-shaped structure, with the apex facing the apex 23 and connected to the main upright 211, and with upper ends connected to the annular edge 212. Taking a main upright 211 as a reference, the pairs 215', 215", 215‴ of secondary uprights are arranged in series, from the smallest 215' to the largest 215'''. In particular, the smaller pair 215' is arranged inside the larger pair 215".

In the example in which the cells 214 of the annular edge 212 are rhomboidal or V- or U-shaped, the main uprights 211 and/or secondary uprights 215 depart from the apex of the cell.

The frame 21 is therefore characterized by a branched structure, provided with greater flexibility at the apex 23. The main uprights 211 are the main traction points of the frame 21 and allow the membrane 22 to be fastened. The secondary uprights 215, on the other hand, allow the tensile forces to be uniformly distributed from the apex 23 to the annular edge 212 and therefore confer uniformity of narrowing in the step of recapturing the temporary valve, at the end of the procedure.

Preferably, the membrane 22 is internally fastened to the frame 21, proximally at the main uprights 211, for example by means of adhesive, or by stitching.

Preferably, the membrane 22 is also connected to the frame 21 at the apex 23 to prevent the passage of blood flow at that point of the temporary valve 2.

Preferably, the membrane 22 is composed of leaflets 221, preferably three triangular leaflets.

The membrane 22 may be made of any thin, flexible and pliable material which is generally impermeable to flow and is biocompatible and non-thrombogenic. Examples of suitable synthetic polymers and copolymers include, but are not limited to, polyester urethane, polyether urethane and polycarbonate urethane with added silicone. The membrane 22 may also consist of a membrane of animal origin, or of a synthetically generated membrane. In one embodiment, the membrane 22 is coated with an antithrombotic material to further enhance biocompatibility. This antithrombotic material may be a natural molecule, such as heparin, or a synthetic one, such as polypyrrole.

It should be noted that in the temporary valve 2 according to the present invention the uprights 211,215 are fastened (i.e. they do not move during operation) and keep the valve in position against the heart wall, while the membrane 22 moves in response to the pumping of the heart to alternately block and allow blood flow through the temporary valve 2, as seen in Fig. 3 and 4.

As may be seen in Fig. 1, the device 1 comprises a sheath 4 slidably mounted along the support body 3 to cover the temporary valve 2 to keep it in a collapsed configuration during the movement of the aorta through the body lumen up to the point of use. Withdrawing the sheath 4 releases the temporary valve 2.

The sheath 4 therefore comprises an internal lumen 41.

Preferably, the sheath 4 completely covers the temporary valve 2.

The sheath consists of a tube of plastics material, coated with a hydrophilic material to facilitate advancement through the blood vessels. The tube has a length of between 80 and 120 cm, a maximum outer diameter of 7mm and a maximum inner diameter of 6mm. The tube is provided proximally with a hemostatic valve to prevent the patient's blood from flowing out, but at the same time allowing the insertion of the temporary valve. Before being inserted into the patient, a dilator is inserted inside the sheath. The dilator consists of a full tube longer than the sheath, the diameter of which tube tends to decrease in the part that comes out of the sheath. This configuration makes it possible to facilitate the advancement of the sheath without causing damage to the blood vessels. The dilator is removed once the sheath has reached the desired position and is replaced by the temporary valve.

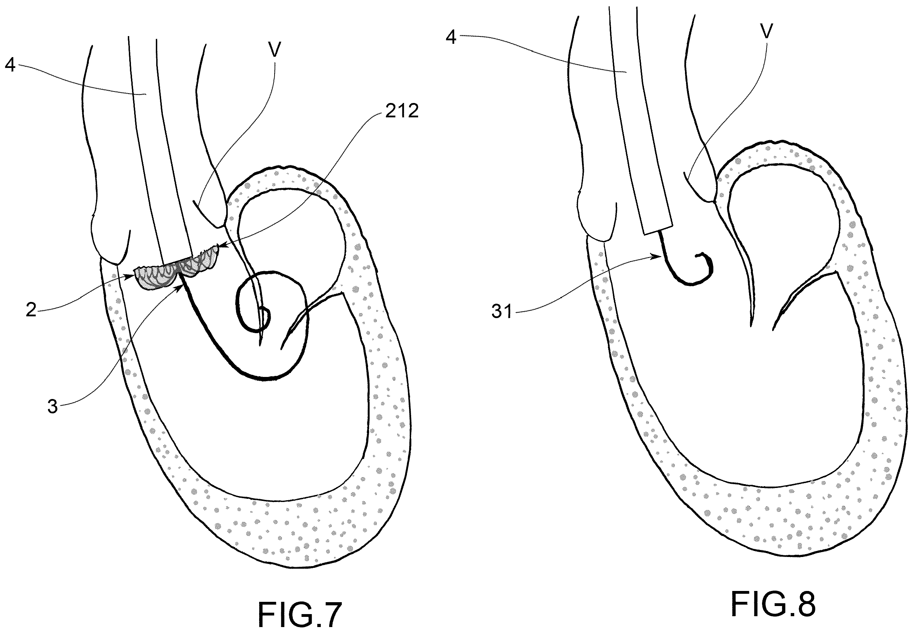

The sheath 4 is also used to recompress the temporary valve 2 and allow the removal thereof at the end of operations, as shown in Fig. 5 to 8. The sheath 4 is first made to advance along the support body 3 to bring it closer to the apex 23 of the temporary valve 2 (Fig. 5). By keeping the sheath 4 in position, the support body 3 is moved back and with it the temporary valve 2 fastened to it at the apex 23. By continuing to withdraw the support body 3, the apex 23 is dragged inside the lumen 41 of the sheath 4 and with it also the frame 21 and the membrane 22 (Fig. 6). It should be noted that the temporary valve 2, during reinsertion into the sheath 4, is turned inside out. The term turned inside out means that, at the end of the withdrawal, what was arranged externally (i.e. the frame 21) is inside and what was arranged internally (i.e. the membrane 22) is outside. By continuing to withdraw the support body 3, the main uprights 211 first and then the secondary uprights 215 are withdrawn inside the lumen 41 of the sheath and lastly the annular edge 212 is also withdrawn (Fig. 7). By withdrawing further, also the flexible tip 31 and therefore the entire support body 3 is completely withdrawn inside the lumen 41 of the sheath (Fig. 8).

To facilitate the withdrawal of the temporary valve 2 by turning it inside out, the frame 21 comprises, at the apex 23, at least one hinge 231.

Preferably, as shown in Fig. 12 and 13, the main uprights 211 are connected to the apex 23 via a hinge 231. In the example of Fig. 13, the hinge 231 is directly connected to the apex 23; in the example of Fig. 12, the hinge 231 is connected to the apex 23 via a pair of flexible bridges 232. It should be noted that the hinge 231 may be made in various ways, for example it is a separate element from the main uprights 211 and pivoted to them, or the hinge 231 is made in one piece with the main uprights 211 with two flexible bridges 232 or a zigzag one.

As shown in Fig. 9 and 10, the support body 3 serves as a guide wire for further devices, such as for example for a delivery catheter 5 of a replacement valve 51.

The device 1 according to the present invention allows procedures to be performed on natural or prosthetic heart valves. In particular, the device 1 is provided with a temporary valve 2 fastened on a support body 3 which is itself a ventricular guide (guide wire). The device 1, and in particular the flexible tip 31, is positioned just below the natural valve or the first TAV, therefore inside the left ventricle in an area called the outflow tract of the left ventricle, as in Fig. 1. By withdrawing the sliding sheath 4, the temporary valve 2 is released in the operating configuration, i.e. open and placed against the outflow tract walls of the left ventricle, as in Fig. 2. At this point, by virtue of the support body 3, which acts as a rail, a delivery catheter 5 of a replacement valve 51 (TAV - Transcatheter Aortic Valve) is fitted and made to slide as shown in Fig. 9. Once the delivery catheter 5 reaches the pathological natural valve or the first damaged TAV, the new valve 51 is released inside the previous valve.

However, in the specific case of the TAVR procedure, before being able to release the second TAV it may be necessary to cut or eliminate the leaflets (leaflet removal, leaflet resection) of the first TAV, since they may prevent correct coronary perfusion or in any case make any type of subsequent coronary intervention difficult. In the period between the removal or cutting of the leaflets of the first TAV and the implantation of the second TAV inside the first, the patient is protected from the risk of aortic regurgitation by virtue of the temporary valve 2 which plays the role of the previous aortic artery during the cutting/removal of the leaflets. At the end of the procedure, the temporary valve 2 is withdrawn as already explained in relation to Fig. 5 to 8.

Innovatively, a device 1 with a temporary valve 2 according to the present invention is easy to use, safe and particularly effective.

Advantageously, the shape and positioning of the temporary valve 2 on the support body 3 in the form of a guide wire allows the second TAV to be implanted without interfering with it.

Advantageously, the temporary valve 2 turns inside out during the withdrawal step so as to be recovered without interfering with the newly implanted TAV.

It is understood that a person skilled in the art could make modifications to the product described above, all of which are contained within the scope of protection as defined by the following claims.

## Claims

1. A device (1) with a temporary valve for percutaneous operations on a natural or artificial aortic valve, comprising:
- a support body (3) distally provided with a flexible tip (31);
- a temporary umbrella valve (2), provided with a distal apex (23) at which it is fastened to the support body (3), said temporary valve (2) having a frame (21) with a membrane (22), wherein said frame (21) allows the temporary valve (2) to pass from a collapsed insertion configuration to an operating configuration, and said membrane (22) allows the temporary valve (2) to pass into the operating configuration between the closed blood flow state and the open blood flow state;
- a sheath (4) sliding along the support body (3) to keep the temporary valve (2) in a collapsed configuration; **characterized in that** the frame (21) comprises a proximal edge (212) from which main uprights (211) start that are connected to each other at the apex (23) and **in that** said frame (21) allows the temporary valve (2) to pass from the operating configuration to a collapsed extraction configuration in which the temporary umbrella valve (2) is turned inside out.

2. The device (1) according to claim 1, wherein the main uprights (211) have increasing flexibility towards the apex (23).

3. The device (1) according to claim 1 or 2, wherein the main uprights (211) have a tapered section towards the apex (23).

4. The device (1) according to any one of the preceding claims, wherein the main uprights (211) are connected to the apex (23) by at least one hinge (231).

5. The device (1) according to any one of the preceding claims, wherein the frame (21) comprises a plurality of secondary uprights (215) starting from the edge (212) and connecting to a main upright (211).

6. The device (1) according to claim 5, wherein the secondary uprights (215) are thinner and/or more flexible with respect to the main uprights (211).

7. The device (1) according to claim 5 or 6, wherein
the secondary uprights (215) are connected to the relevant main upright (211) with an offset trend between right and left, or
the secondary uprights (215) are arranged in pairs, with a first secondary upright arranged on one side of the main upright (211), and a second secondary upright arranged specularly on the opposite side of the main upright (211).

8. The device (1) according to any one of the preceding claims, wherein the membrane (22) is internally fastened to the frame (21), proximally at the main uprights (211) and distally at the apex (23).

9. The device (1) according to any one of the preceding claims, wherein the flexible tip (31) is preformed and provided with a final curvature or curl (31').

10. The device (1) according to any one of the preceding claims, wherein the edge (12) is formed by
closed rhomboid cells (214) connected to each other at the smaller diagonal, or
V or U-shaped open cells (214), with the vertex facing the apex (23) and connected to each other at the upper ends.

## Patentansprüche

1. Vorrichtung (1) mit einer provisorischen Klappe für perkutane Operationen an einer natürlichen oder künstlichen Aortenklappe, umfassend:
- einen Stützkörper (3), der distal mit einer flexiblen Spitze (31) versehen ist;
- eine provisorische Schirmklappe (2), die mit einem distalen Scheitel (23) versehen ist, an dem sie an dem Stützkörper (3) befestigt ist, wobei die provisorische Klappe (2) einen Rahmen (21) mit einer Membran (22) aufweist, wobei der Rahmen (21) es der provisorischen Klappe (2) ermöglicht, von einer zusammengeklappten Einführkonfiguration in eine Betriebskonfiguration überzugehen, und wobei die Membran (22) es der provisorischen Klappe (2) ermöglicht, in der Betriebskonfiguration zwischen dem Zustand mit geschlossenem Blutfluss und dem Zustand mit offenem Blutfluss zu wechseln;
- eine Hülse (4), die entlang des Stützkörpers (3) gleitet, um die provisorische Klappe (2) in einer zusammengeklappten Konfiguration zu halten;
**dadurch gekennzeichnet, dass** der Rahmen (21) einen proximalen Rand (212) umfasst, von dem Hauptstreben (211) ausgehen, die am Scheitel (23) miteinander verbunden sind, und dadurch, dass der Rahmen (21) es der provisorischen Klappe (2) ermöglicht, von der Betriebskonfiguration in eine zusammengeklappte Extraktionskonfiguration überzugehen, in der die provisorische Schirmklappe (2) von innen nach außen umgestülpt ist.

2. Vorrichtung (1) nach Anspruch 1, wobei die Hauptstreben (211) zum Scheitel (23) hin eine zunehmende Flexibilität aufweisen.

3. Vorrichtung (1) nach Anspruch 1 oder 2, wobei die Hauptstreben (211) zum Scheitel (23) hin einen verjüngten Querschnitt aufweisen.

4. Vorrichtung (1) nach einem der vorhergehenden Ansprüche, wobei die Hauptstreben (211) durch mindestens ein Scharnier (231) mit dem Scheitel (23) verbunden sind.

5. Vorrichtung (1) nach einem der vorhergehenden Ansprüche, wobei der Rahmen (21) eine Vielzahl von Sekundärstreben (215) umfasst, die vom Rand (212) ausgehen und mit einer Hauptstrebe (211) verbunden sind.

6. Vorrichtung (1) nach Anspruch 5, wobei die Sekundärstreben (215) in Bezug auf die Hauptstreben (211) dünner und/oder flexibler sind.

7. Vorrichtung (1) nach Anspruch 5 oder 6, wobei die Sekundärstreben (215) mit der entsprechenden Hauptstrebe (211) mit einem versetzten Verlauf zwischen rechts und links verbunden sind, oder
die Sekundärstreben (215) paarweise angeordnet sind, wobei eine erste Sekundärstrebe auf einer Seite der Hauptstrebe (211) angeordnet ist und eine zweite Sekundärstrebe spiegelbildlich auf der gegenüberliegenden Seite der Hauptstrebe (211) angeordnet ist.

8. Vorrichtung (1) nach einem der vorhergehenden Ansprüche, wobei die Membran (22) innen am Rahmen (21) befestigt ist, und zwar proximal an den Hauptstreben (211) und distal am Scheitel (23).

9. Vorrichtung (1) nach einem der vorhergehenden Ansprüche, wobei die flexible Spitze (31) vorgeformt und mit einer Endkrümmung oder Windung (31') versehen ist.

10. Vorrichtung (1) nach einem der vorhergehenden Ansprüche, wobei der Rand (12) gebildet wird durch
geschlossene rautenförmige Zellen (214), die an der kleineren Diagonale miteinander verbunden sind, oder
V- oder U-förmige offene Zellen (214), deren Spitze dem Scheitel (23) zugewandt ist und die an den oberen Enden miteinander verbunden sind.

## Revendications

1. Dispositif (1) avec une valve temporaire pour les opérations percutanées sur une valve aortique naturelle ou artificielle, comprenant :
- un corps de support (3) muni de façon distale d'une pointe flexible (31) ;
- une valve parapluie temporaire (2), munie d'un apex distal (23) au niveau duquel elle est fixée au corps de support (3), ladite valve temporaire (2) ayant un cadre (21) avec une membrane (22), dans lequel ledit cadre (21) permet à la valve temporaire (2) de passer d'une configuration d'insertion repliée à une configuration de fonctionnement, et ladite membrane (22) permet à la valve temporaire (2) de passer dans la configuration de fonctionnement entre l'état de flux sanguin fermé et l'état de flux sanguin ouvert ;
- une gaine (4) coulissant le long du corps de support (3) pour maintenir la valve temporaire (2) dans une configuration repliée ;
**caractérisé en ce que** le cadre (21) comprend un bord proximal (212) à partir duquel partent les montants principaux (211) qui sont reliés les uns aux autres au niveau de l'apex (23) et **en ce que** ledit cadre (21) permet à la valve temporaire (2) de passer de la configuration de fonctionnement à une configuration d'extraction repliée dans laquelle la valve parapluie temporaire (2) est retournée.

2. Dispositif (1) selon la revendication 1, dans lequel les montants principaux (211) présentent une flexibilité croissante vers l'apex (23).

3. Dispositif (1) selon la revendication 1 ou 2, dans lequel les montants principaux (211) présentent une section effilée vers l'apex (23).

4. Dispositif (1) selon l'une quelconque des revendications précédentes, dans lequel les montants principaux (211) sont reliés à l'apex (23) par au moins une charnière (231).

5. Dispositif (1) selon l'une quelconque des revendications précédentes, dans lequel le cadre (21) comprend une pluralité de montants secondaires (215) partant du bord (212) et se raccordant à un montant principal (211).

6. Dispositif (1) selon la revendication 5, dans lequel les montants secondaires (215) sont plus minces et/ou plus flexibles par rapport aux montants principaux (211).

7. Dispositif (1) selon la revendication 5 ou 6, dans lequel les montants secondaires (215) sont reliés au montant principal concerné (211) avec une tendance de décalage entre la droite et la gauche, ou
les montants secondaires (215) sont disposés par paires, avec un premier montant secondaire disposé sur un côté du montant principal (211), et un deuxième montant secondaire disposé de manière spéculaire sur le côté opposé du montant principal (211).

8. Dispositif (1) selon l'une quelconque des revendications précédentes, dans lequel la membrane (22) est fixée intérieurement au cadre (21), de façon proximale au niveau des montants principaux (211) et de façon distale au niveau de l'apex (23).

9. Dispositif (1) selon l'une quelconque des revendications précédentes, dans lequel la pointe flexible (31) est préformée et pourvue d'une courbure ou d'une boucle (31') finale.

10. Dispositif (1) selon l'une quelconque des revendications précédentes, dans lequel le bord (12) est formé par
des cellules rhomboïdales fermées (214) reliées les unes aux autres au niveau de la plus petite diagonale, ou
des cellules ouvertes en forme de V ou de U (214), le sommet faisant face à l'apex (23) et étant reliées les unes aux autres au niveau des extrémités supérieures.
